(19) Europäisches Patentamt
European Patent Office
Office européen des brevets

(11) **EP 3 852 039 A1**

(12) **EUROPEAN PATENT APPLICATION**
published in accordance with Art. 153(4) EPC

(43) Date of publication:
**21.07.2021 Bulletin 2021/29**

(51) Int Cl.:
**G06Q 20/06** *(2012.01)*    **G06Q 20/38** *(2012.01)*

(21) Application number: **19876253.6**

(86) International application number:
**PCT/JP2019/040233**

(22) Date of filing: **11.10.2019**

(87) International publication number:
**WO 2020/085125 (30.04.2020 Gazette 2020/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **25.10.2018 JP 2018200583**

(71) Applicant: **MAZDA MOTOR CORPORATION**
**Hiroshima 730-8670 (JP)**

(72) Inventor: **TOCHIOKA Takahiro**
**Aki-gun, Hiroshima 730-8670 (JP)**

(74) Representative: **Behr, Wolfgang**
**Lorenz Seidler Gossel**
**Rechtsanwälte Patentanwälte**
**Partnerschaft mbB**
**Widenmayerstraße 23**
**80538 München (DE)**

(54) **VIRTUAL CURRENCY MANAGEMENT DEVICE AND VIRTUAL CURRENCY MANAGEMENT METHOD**

(57)     A virtual currency management device 50 is configured to: estimate a well-being value (WB value) as a mental activity level of a user A who participates in a give event E; store an amount of legal currency spent in the event E by the user A; generate virtual currency based on the estimated well-being value; store and manage the generated virtual currency in association with the user A; perform processing for selling the managed virtual currency to an investor I (third party), and buying out the virtual currency of the investor I; and set a conversion rate between the virtual currency and the legal currency to be used when the investor I buys or sells the virtual currency, such that the monetary value of the virtual currency increases relative to the legal currency as the stored amount of the legal currency is larger.

FIG.6

**Description**

TECHNICAL FIELD

The present invention relates to a system and method for managing virtual currency.

BACKGROUND ART

In late years, as well as transactions for goods using legal currency such as yen or dollar, commercial transactions using virtual currency such as bitcoin have been performed. Differently from legal currency or electronic money, virtual currency means a currency used under a system in which various commercial transactions are performed by using mining programs linked to each other via a network, and thereby security of the transactions is ensured.

Techniques relating to such virtual currency are disclosed in, e.g., the below-mentioned Patent Documents 1 and 2. In the Patent Document 1, there is disclosed a technique for use in a virtual currency management device for performing conversion between legal currency and virtual currency, wherein the technique is intended to stabilize the monetary value of the virtual currency, i.e., the rate of the virtual currency with respect to the legal currency. Further, in the Patent Document 2, there is disclosed a technique of, after buying virtual currency, gradually diminishing the monetary value of the virtual currency over time.

CITATION LIST

[Parent Document]

Patent Document 1: JP-B 6352463

Patent Document 2: JP-B 5871347

SUMMARY OF INVENTION

Meanwhile, in recent years, researches on "well-being" have been conducted. The term "well-being" does not mean that a person is merely healthy (not sick), but means that a person is in physically, mentally and societally good states.

In a technical field relating to the virtual currency as mentioned above, there has heretofore been no technique of providing a service while directly associating well-being with virtual currency. For example, there has been no service of providing an event intended to motivate a user to perform an activity to enhance the level of well-being, and giving, to the user, virtual currency in an amount according to the level of well-being during this event. If a third party other than the user (i.e., a person or group who does not participate in the event and thus does not generate any virtual currency by itself) can adequately perform transactions for the virtual currency generated based on well-being in the above service, the service is considered to become better. Further, assuming such a service, it is necessary to further consider how to set a conversion rate between virtual currency and legal currency when the third party sells/buys the virtual currency.

The present invention has been made to solve the above problem, and an object of the present invention to provide a virtual currency management device and a virtual currency management method capable of, when performing, with a third party, transactions for virtual currency generated according to a well-being value of a user who participates in an event, adequately setting a conversion rate between the virtual currency and legal currency.

[Solution to Technical Problem]

In order to achieve the above object, according to one aspect of the present invention, there is provided a virtual currency management device comprising at least a computer. The virtual currency management device is configured to: estimate a well-being value as a mental activity level of a user who participates in a give event; store an amount of legal currency spent in the event by the user; generate virtual currency based on the estimated well-being value; store and manage the generated virtual currency in association with the user; perform processing for selling the managed virtual currency to a third party, and buying out the virtual currency of the third party; and set a conversion rate between the virtual currency and the legal currency to be applied when the third party buys or sells the virtual currency, such that a monetary value of the virtual currency increases relative to the legal currency as the stored amount of the legal currency is larger.

In the present invention having the above feature, the virtual currency management device estimates the well-being value of a user who participates in a give event to generate virtual currency based on the estimated well-being value, and sells/buys the virtual currency to/from a third party (investor). This makes it possible to adequately perform transactions for the virtual currency generated by the user who participates in the event, with the third party. In particular, this makes it possible for the third party to adequately invest in such virtual currency. Therefore, it becomes possible to encourage the user or the like to participate in the event, and encourage a consumption behavior in the event, through buying of the virtual currency (investment) by the third party, and thereby realize activation of the event, activation of an area in which the event is held, etc.

Particularly, the virtual currency management device of the present invention sets the conversion rate between the virtual currency and the legal currency, so as to cause the monetary value of the virtual currency to be increased as the amount of legal currency spent in the event by the user is larger. That is, based on the assumption that a user who spent a large amount of legal currency in the event has a strong tendency to become a well-being state, the monetary value of virtual currency generated by such a user can be adequately raised. Therefore, the present invention makes it possible to, when performing, with the third party, transactions for the virtual currency generated by the user who participates in the event, adequately set the conversion rate between the virtual currency and the legal currency.

Preferably, in the virtual currency management device of the present invention, the conversion rate is set based on a cumulative amount or average amount of the legal currency spent in a plurality of events by the user.

According to this feature, it becomes possible to set the virtual currency conversion rate, while adequately taking into account an overall tendency for the user to spend the legal currency in the plurality of events.

Preferably, the virtual currency management device of the present invention is configured to store an estimation accuracy of the well-being value, and set the conversion rate such that the monetary value of the virtual currency increases relative to the legal currency as the stored estimation accuracy of the well-being value is higher.

According to this feature, based on the assumption that a user who willingly takes a behavior for estimating the well-being value in an event, i.e., a user who is cooperative in estimating the well-being value is considered to have a strong tendency to become the well-being state, the monetary value of virtual currency generated by such a user can be adequately raised.

More preferably, in the above virtual currency management device, the conversion rate is set based on an average of the estimation accuracy of the well-being value estimated in a plurality of events.

According to this feature, it becomes possible to set the virtual currency conversion rate while adequately taking into account an overall tendency of the estimation accuracy of the well-being value in the plurality of events.

More preferably, the above virtual currency management device is configured to be capable of acquiring a plurality of types of data, for the estimation of the well-being value, and determine that the estimation accuracy of the well-being value is higher as the number of the types of data actually used in the estimation of the well-being value among the plurality of types of data is larger.

According to this feature, it becomes possible to adequately determine the estimation accuracy of the well-being value.

More preferably, the above virtual currency management device is configured to be capable of acquiring, as the plurality of types of data, one or more pieces of biological data indicative of a physiological state of the user during event participation, emotion data based on voice made by the user during event participation, and evaluation data which is a level of a subjective evaluation made by the user for each event.

According to this feature, it becomes possible to accurately estimate the well-being value, using the biological data, the emotion data and the evaluation data of the user.

More preferably, the above virtual currency management device is configured to: estimate the well-being value, based on at least one selected from the group consisting of one or more pieces of biological data indicative of a physiological state of the user during event participation, emotion data based on voice made by the user during event participation, and evaluation data which is a level of a subjective evaluation made by the user for each event, and determine that the estimation accuracy of the well-being value estimated based on the evaluation data is higher than the estimation accuracy of the well-being value estimated based on the emotion data or the biological data.

According to this feature, it becomes possible to adequately determine the estimation accuracy of the well-being value.

Preferably, the virtual currency management device of the present invention is configured to set the conversion rate, with respect to each virtual currency of a single user, or with respect to each virtual currency of a given group including a plurality of users.

According to this feature, it becomes possible to adequately change the virtual currency conversion rate, according to credibility (monetary value) of the virtual currency of each single user or each given group.

In order to achieve the above object, according to another aspect of the present invention, there is provided a virtual currency management method executed by a computer. The method comprising the steps of: estimating a well-being value as a mental activity level of a user who participates in a give event; storing an amount of legal currency spent in the event by the user; generating virtual currency based on the estimated well-being value; storing and managing the generated virtual currency in association with the user; performing processing for selling the managed virtual currency to a third party, and buying out the virtual currency of the third party; and setting a conversion rate between the virtual currency and the legal currency to be applied when the third party buys or sells the virtual currency, such that a monetary value of the virtual currency increases relative to the legal currency as the stored amount of the legal currency is larger.

The present invention having the above feature also makes it possible to, when performing, with a third party, transactions for the virtual currency generated by the user who participates in the event, adequately set the conversion rate between the virtual currency and the legal currency.

[Effect of Invention]

The virtual currency management device and the virtual currency management method of the present invention make it possible to, when performing, with a third party, transactions for the virtual currency generated in an amount according to the well-being value of the user who participates in the event, adequately set the conversion rate between the virtual currency and the legal currency.

BRIEF DESCRIPTION OF DRAWINGS

FIG. 1 is a conceptual diagram of a life activation system according to one embodiment of the present invention.
FIG. 2 is a configuration diagram of the life activation system according to this embodiment.
FIG. 3A is a graph showing a temporal change in WB value.
FIG. 3B is a graph showing a temporal change in the balance of virtual currency of a user in this embodiment.
FIG. 4 is an explanatory diagram of estimation of the WB value in an activity in this embodiment.
FIG. 5 is a processing flow for estimating the WB value in the activity in this embodiment.
FIG. 6 is a conceptual diagram of a virtual currency transaction system according to another, second, embodiment of the present invention.
FIG. 7 is a schematic configuration diagram of a virtual currency management device in the second embodiment.
FIG. 8 is a processing flow for performing conversion between virtual currency and legal currency in the second embodiment.

DESCRIPTION OF EMBODIMENTS

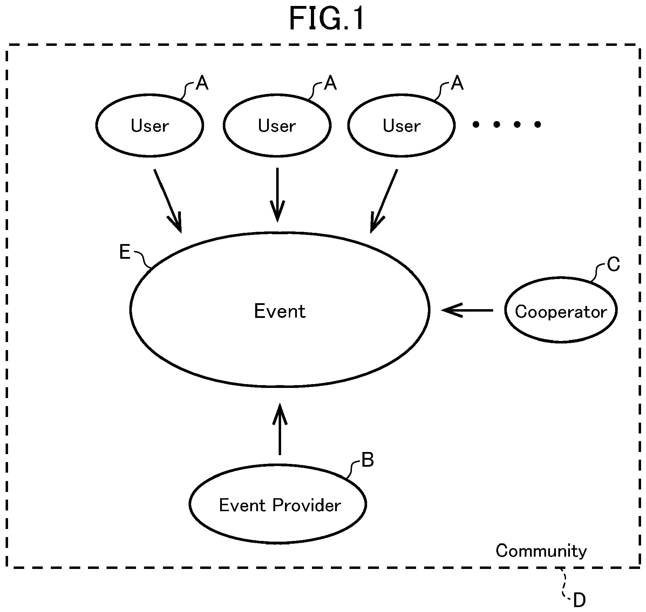

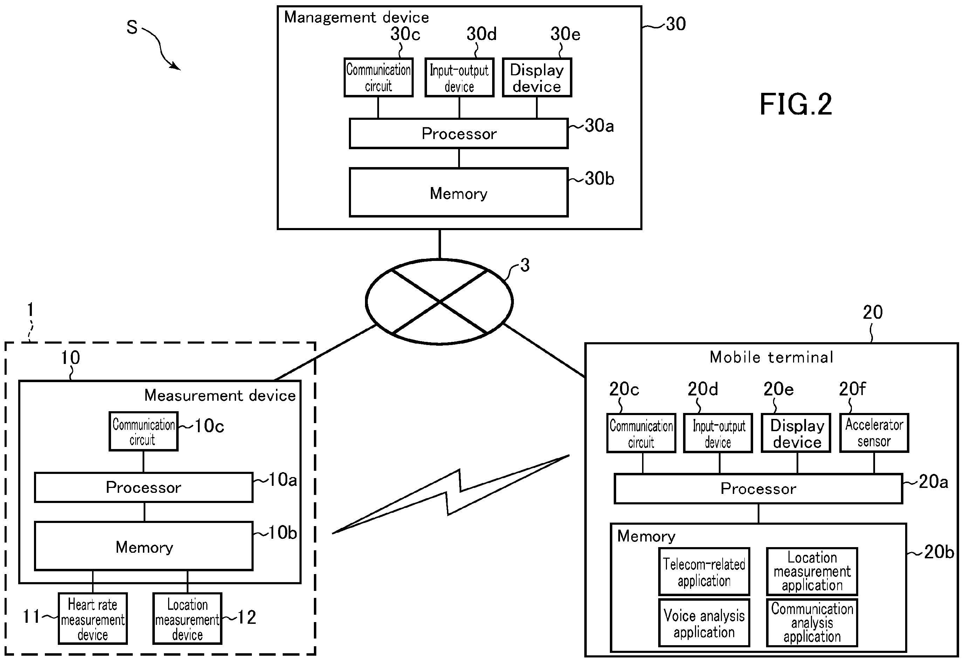

With reference to the drawings, a life activation system according to one embodiment of the present invention will now be described. First of all, a schematic configuration of the life activation system will be described with reference to FIGS. 1 and 2. FIG. 1 is a conceptual diagram of the life activation system, and FIG. 2 is a configuration diagram of the life activation system. In the following description, the "life activation" and the "well-being" will be occasionally abbreviated, respectively, as "LA" and "WB".

As used in this embodiment, the term "well-being" means that a user overcomes his/her self-denying negative emotions, and achieves a feeling of self-fulfillment and a feeling of self-affirmation in relation with society (other people). The feeling of self-affirmation includes feeling that the existence of the user is needed in and useful for society.

Further, the term "life activation" means realizing a life active state in which the user is engaged with society through life with an automotive vehicle (possession/driving of the vehicle), to continuously perform an activity (WB activity) of increasing the level of the well-being (WB value). Then, through life in the life active state, the user will accumulate WB activities or WB experiences. This process can be defined as "well-aging". In this case, continuous accumulation of WB experiments is deemed to allow the user to reach a higher-order WB state.

The LA system according to this embodiment is configured such that an economic value (virtual currency) is given to a user's action of participating in an event to perform a WB activity. This virtual currency can be exchanged for legal currency at a given exchange rate. Thus, through participation in the event, it is possible to increase the WB value of the user in physical, mental and societal aspects, and give, to the user, an incentive to participate an event in an economic aspect.

As shown in FIG. 1, the life activation system S (LA system S) according to this embodiment is a system for allowing a user A to increase the WB value of the user A, through participation in an event E. Examples of the event E include a sightseeing tour, a nature tour, a mystery tour, and a volunteer activity.

An event provider B organizes and realizes the event E under personnel and financial cooperation of a cooperator C. Examples of the cooperator C include a company and community people in an event venue, and a funder. The user A pays out various expenses (participation fee, gasoline expense, meal expense, etc.) in connection with the event E.

The event provider B and the cooperator C can recover an operating expense by a spend from the user A. The LA system according to this embodiment is configured such that a community D is formed of the users A, the event provider B and the cooperator C, through the event E, wherein funds are circulated within the community D to allow various events to be repeatedly held.

Next, with reference to FIG. 2, the LA system S according to this embodiment will be described. The LA system S comprises a measurement device 10 mounted to a vehicle 1 of a user A, a mobile terminal 20 of the user A, and a management device 30 of an event provider B, wherein these components are configured to be wirelessly communicable with each other. Each of the measurement device 10 and the mobile terminal 20 is wirelessly communicable with the management device 30 via a communication line (Internet line) 3. The measurement device 10 and the mobile terminal 20 are communicable with each other by means of a near field communication technique (e.g., Bluetooth (registered trademark)).

The measurement device 10 is composed of a computer device comprising a processor 10a, a memory 10b, and a communication circuit 10c, and configured to cause the processor 10a to execute various programs stored in the memory 10b, thereby performing processings (including a plurality of analytical processings). The measuring device 10 is connected to various sensor devices mounted to the vehicle 1, wherein the processor 10a is operable to receive measured data from the sensor devices and store the measured data in the memory 10b. Then, the processor 10a is operable to process the measured data, and transmit the resulting processed data to the management device 30 through the communication circuit 10c.

The sensor device comprises a heart rate measurement device 11 attached to a steering wheel, and a location measurement device 12. The heart rate measurement device 11 is one example of a biological sensor device. The heart rate measurement device 11 is operable to measure a heart rate variability (electrocardiographic waveform) of a driver (user A) and output the resulting measured data as biological data (heart-rate data). The location measurement device 12 is operable to acquire a vehicle location by means of satellite positioning using the GPS (Global Positioning System), or autonomous positioning using a gyrosensor or the like.

It should be noted here that a resistance measurement device or a camera may be used as the biological sensor device, in place of or in addition to the heart rate measurement device 11. Specifically, the resistance measurement device is operable to measure a skin resistance of the user A, and output the resulting measured data as biological data (resistance data). A contact resistance with skin varies depending on a sweating state, and therefore the resistance data indicates the sweating state of the user A. The camera is operable to capture an image of the user A, and output data about captured image. In this case, the measurement device 10 is operable to subject this image data to image analysis to acquire biological data, such as a pupil diameter and an eye movement of the user A, and the behavior of the upper body of the user A including the position and orientation of the head or shoulder, or the emotion of the face of the user A.

Generally, in a tense state in which a person feels mental stress, the person undergoes a situation where: heart rate variability occurs; sweating is promoted; the pupil diameter is enlarged; and the range of eye movement is expanded. Further, in the tense state, the person undergoes a situation where the behavior of the upper body including the position and orientation of the head or shoulder becomes larger, during vehicle acceleration/deceleration or turning. Further, in the tense state, the person undergoes a situation where a change appears in emotion of the face (movement distance of a specific area of the face, etc.).

Thus, the measurement device 10 is operable to estimate a psychological (mental) state (i.e., tension (stress) level) of the user by analyzing the above biological data. For example, the measurement device 10 is operable to estimate the tension level (e.g., which ranges from 0 (lowest level) to 10 (highest level)), based on the magnitude of a measured value of the biological data (the frequency obtained by subjecting the heart rate variability to frequency analysis, the skin resistance, the pupil diameter, the eye movement, the behavior of the upper body, the movement distance of a specific area of the face). When the tension level is at an optimal medium level (e.g., tension level = 5), the user A is determined to be in a mentally good active state.

The measurement device 10 is operable to store, in the memory 10b, data about a result of the analysis of the mental state estimated based on the biological data, and the vehicle location data, as needed basis, and transmit these data to the management device 30 together with vehicle identification data identifying the vehicle 1.

In this embodiment, the measurement device 10 is mounted to the vehicle 1. Alternatively, the measurement device 10 may be configured as a mobile biological information measurement device so as to allow the user A to always carry it. For example, the measurement device 10 may be a wristband-type heart rate measurement device with a communication function. In this case, the measurement device 10 is operable to always transmit the mental state analysis result data to the management device 30.

The mobile terminal 20 is a mobile computer device comprising a processor 20a, a memory 20b, a communication circuit 20c, an input-output device 20d, a display device 20e, and an accelerator sensor 20f. The mobile terminal 20 is one example of a wearable sensor terminal device. The mobile terminal 20 is configured to execute by the processor 20a various programs stored in the memory 20b, thereby performing various processings. In this embodiment, the mobile terminal 20 is composed of a smartphone, so that it has a voice communication function and a data communication function which are originally equipped in the smartphone.

The mobile terminal 20 is also configured to execute various applications (application programs) installed in the memory 20b, thereby functioning as various sensor terminal devices. The processor 20a is operable to transmit and receive a variety of data with respect to the management device 30 through the communication circuit 20c. Examples of the variety of data include analysis result data to be transmitted by the mobile terminal 20, and commands to be transmitted by the management device 30.

The input-output device 20d comprises a key board or touch sensor-type input device, a microphone, and a speaker. It should be understood that the mobile terminal 20 may be a mobile information terminal such as a personal digital assistance (PDA), or may be a dedicated small-size computer device.

The mobile terminal 20 also functions as a communication device through the use of a telecom-related application. Specifically, the mobile terminal 20 has a call function, an e-mail function, an SNS communication function (e.g., Facebook (registered trademark), Instagram (registered trademark) or LINE (registered trademark)), and a browser function for accessing websites via the Internet line 3.

Further, the mobile terminal 20 functions as a voice analysis device through the use of a voice analysis application. The mobile terminal 20 is operable to analyze a voice spoken by the user A for a given time period (e.g., for 2 to 6 seconds) to estimate an emotional state of the user.

A voice spoken by a person contains a voluntary element which can be controlled by the person, and a non-voluntary element relating to emotion of the brain. More specifically, the vocal cords connect to a region of the brain responsible for its emotion via the vagus nerve, so that the emotion of the brain exerts an influence on the movement of the vocal cords. Thus, the emotion of the brain exerts an influence on vocal parameters (fundamental frequency, sound pressure, pitch frequency, frequency distribution, speech rate, vocal intensity variation, etc.).

In this embodiment, the mobile terminal 20 stores, in the memory 20b, a table indicating a correlative relationship between a given vocal parameter (in this embodiment, the magnitude of the fundamental frequency, and the intensity at the fundamental frequency) and each emotion (in this embodiment, joy, sadness, ease, anger). The mobile terminal 20 is configured to analyze voice based on this table to estimate the rate of each emotion. The total value of the respective rates of the emotions is constant (e.g., 100%).

The mobile terminal 20 is operable to compare a given vocal parameter (in this embodiment, pitch frequency) with a given value to estimate the level of excitement (excitement levels: e.g., 0 (lowest level) to 10 (highest level)), and multiply the estimated excitement level by the estimated rate of each emotion to estimate the strength of each emotion. Here, the pitch frequency means the frequency of repetition of the same frequency component. As the pitch frequency becomes increasingly greater than the given value, the excitement level becomes higher.

As the mind and body of a person become healthier, the strength of the positive emotion (joy, ease) becomes increasingly greater than the negative emotion (sadness, anger). The mobile terminal 20 is configured to calculate a mental activation level (or mental soundness level) based on, e.g., a value obtained by multiplying the estimated strength of each emotion by a coefficient set for each emotion, and summing the resulting products. For example, the coefficient for the positive emotion may be a positive value (> 0), and the coefficient for the negative emotion may be a negative value (< 0). The mental soundness level may be expressed in the range of 0 (lowest level) to 100 (highest level).

The mobile terminal 20 serving as a voice analysis device is operable to always acquire a voice spoken by the user A through the microphone. Further, the mobile terminal 20 is operable to give, to the user, an inquiry (e.g., "How was the event?") for prompting the user to speak, through the speaker, intermittently (at intervals of a given time period (e.g., 10 minutes), and in response to a commend from the management device 30). Then, the mobile terminal 20 is operable to acquire a voice spoken by the user A in response to the inquiry. The mobile terminal 20 is operable to analyze the acquired voice data, and store a result of the voice analysis (emotion data indicating the emotional state) in the memory 20b together with an acquisition time. It should be noted that this function as the voice analysis device may also be provided in the measurement device 10 of the vehicle 1, in addition to the mobile terminal 20. An application for such voice analysis is provided, e.g., as the product name "MIMOSYS (registered trademark)" from PST Inc.

Further, the mobile terminal 20 functions as a location measurement device through the use of a location measurement application. The mobile terminal 20 is operable, based on communication with surrounding communication stations, or based on a GPS-based satellite positioning system, to acquire a current location of the mobile terminal 20, and store the acquired current location in the memory 20b as current location data. The mobile terminal 20 is operable to transmit the current location data to the management device 30 at a given time. The management device 30 is operable, based on the current location data, to track a movement locus of the user A, thereby generating an action log data.

Further, the mobile terminal 20 functions as a communication analysis device through the use of a communication analysis application. The mobile terminal 20 is operable to subject text data posted by the user A using the e-mail function, the SNS communication function, or the browser function, to natural language processing to analyze the text data (text mining). The mobile terminal 20 may be configured to further analyze a reply (e.g., a return mail, an approval response such as "Like", posted comment, etc.) of a friend or the like in response to posting by the user A.

The mobile terminal 20 is operable to transmit a result of the analysis to the management device 30.

The mobile terminal 20 serving as the communication analysis device is operable to extract a specific word expressing the emotional state ("delightful", "joyful", "sad", "painful", etc.), from text data transmitted or posted by the user, based on an expression table which is a part of the communication analysis application, and analyze the emotional state of the user, from the frequency of appearance of these words, or the like. In the expression table, a large number of expressions are stored, correspondingly to each of the emotions (delight, anger, sorrow and pleasure). Therefore, a result of the communication analysis includes emotion data indicating the emotional state (in this embodiment, joy, sadness, ease, anger) of the user during posting. The emotion data includes the rate and strength of each of the four emotions. It should be noted that the mobile terminal 20 may be configured to analyze the emotional state, from image data transmitted or posted by the user.

Further, the mobile terminal 20 functions as a behavior measurement device through the use of a behavior measurement application. The mobile terminal 20 is operable, when the user moves with the mobile terminal 20, to calculate an acceleration and a jerk, based on measured data received from the acceleration sensor 20f. The calculated acceleration and jerk are stored in the memory 20b as behavior data. The mobile terminal 20 is operable to transmit the behavior data to the management device 30 at a given time.

The management device 30 is composed of a computer device (server device) comprising a processor 30a, a memory 30b, a communication circuit 30c, an input-output device 30d, and a display device 30e, and configured to cause the processor 30a to execute various programs stored in the memory 30b, thereby performing various processings. In addition to programs, the memory 30b stores therein event data about various events to be provided by the event provider. The event data includes information about the schedule, venue, content, participants, etc., of each event. The management device 30 is operable to receive data from the measurement device 10 and the mobile terminal 20 of each participant (user A). The management device 30 is operable, based on the received data and the event data, to transmit given information and commands to the measurement device 10 and the mobile terminal 20.

The memory 30b of the management device 30 stores therein user data of each user A (and each event-related person). The user data includes: personal data (age, gender, address, vehicle identification information, mobile terminal information, etc.), action log data, a variety of data (vehicle location data, emotion data, current location data, behavior data, and estimated WB value), and virtual currency data of each user A. The virtual currency data indicates the amount (sum) of virtual currency owned by each user A.

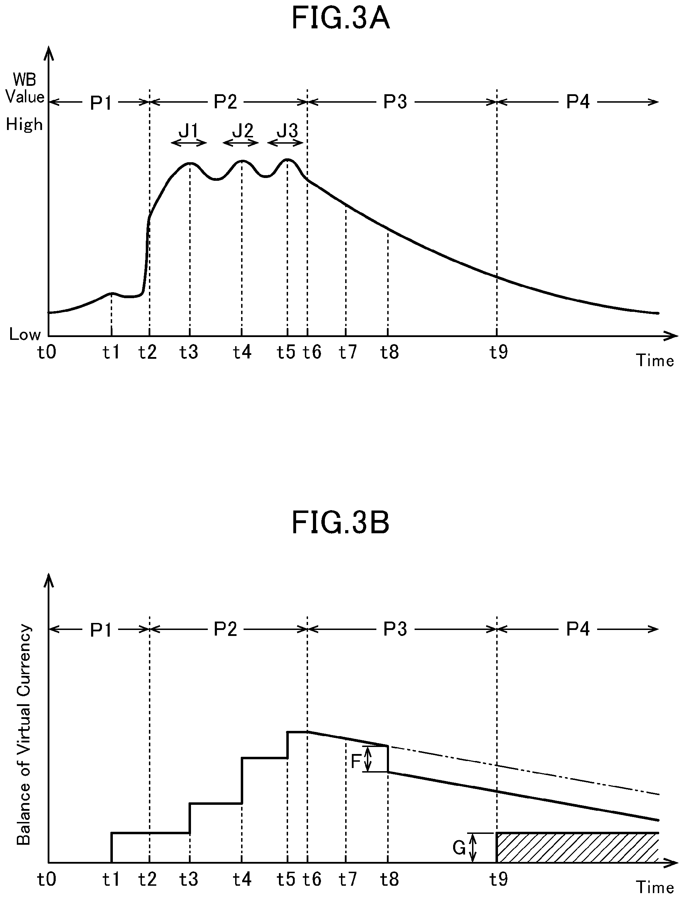

Next, the behavior of a user when participating in an event and a temporal change in the WB value will be described with reference to FIG. 3A. FIG. 3A is a graph showing a temporal change in the WB value. In FIG. 3A, a user A participates in a certain event E (1-day community experience tour). The time period shown in FIG. 3A comprises: a preparation period P1 (e.g., three weeks) before the user A participates in the event E; an action period P2 (e.g., one day) during which the user A participates in the event E; a fostering period P3 (e.g., four weeks) after termination of the event E; and an establishment period P4 (e.g., three months).

The event E is organized to allow the user A to have various experiences through interaction with friends and people met in the event, thereby increasing the WB value of the user A. The user A moves to a venue by using the vehicle 1, and enjoys an event activity (content) in the event E. Then, after termination of the event E, the user A returns home by using the vehicle 1. In a situation where the venue is divided into a plurality of areas, the user A moves from one venue to the next by using the vehicle 1.

Generally, in daily life, it is difficult for the user A to obtain a feeling of WB (i.e., the WB value is at a low level). The user A comes in contact with information about the event E during the preparation period P1 (time t0). The preparation period P1 is a time period during which the user A prepares for participating in the event E which is unordinary, i.e., different from daily life.

When the user A comes in contact with information about the event E, and the mood for wanting to participate in the event E is fostered in the user A, this mood acts to cancel out a self-denying emotion of the user A. Thus, the WB value of the user A rises. The user A in a state in which the WB value thereof becomes higher than a normal value transmits entry information from the mobile terminal 20 to the management device 30 (time t1).

In the preparation period P1, the user A can inform a friend of the participation in the event E, using the communication function (e.g., the SNS communication function, or e-mail function) of the mobile terminal 20. That is, the user A can express a feeling of expectation for the event E, using text data or image data, and post this expression via the Internet line 3. Thus, the feeling of expectation swells in the user A, and a feeling of self-fulfillment (virtually experiencing the event E) is gradually satisfied through the notice announcement to the friend. In this way, in the preparation period P1, the WB value of the user A is maintained to be higher than the normal value. In the preparation period P1, the mobile terminal 20 (communication analysis device) analyzes contents of data posted by the user A and response data, and transmits resulting analysis result data to the management device 30. Further, the mobile terminal 20 (voice analysis device) analyzes a voice of the user A at intervals of a given time period and in response to a command from the management device 30, and transmits resulting analysis result data to the management device 30. The management device 30 can use the received analysis result data for the purpose of estimation of the WB value.

In the action period P2, when the user A drives the vehicle 1 to move toward the event venue (time t2), the self-denying emotion is further canceled, and the feeling of self-fulfillment of the user is gradually satisfied, so that the WB value rises. The management device 30 is operable to receive analysis result data acquired by the measurement device 10 and the mobile terminal 20 (voice analysis device) during driving, and use the received analysis result data for the purpose of estimation of the WB value.

After arrival in the event venue, the user A experiences three main activities (contents) J1, J2, J3 (at times t3, t4, t5; each having a given time length) constituting the event E. In each of the activities (e.g., an experience of making a local specialty, and an experience of harvesting agricultural products), a feeling of self-affirmation stirs in the user A, together with the feeling of self-fulfillment, so that the WB value takes a maximum value. Although the maximized WB value gradually decreases over time after termination of one activity, the WB value is maximized again by the next activity. It should be understood that the event E may have only one activity.

In the action period P2, based on the event data (and, if necessary, the current location data of the user A), the management device 30 transmits individual inquiry information to the mobile terminal 20 of the user A after termination of each activity. This individual inquiry information is intended to check a satisfaction level of the user A with each of the activities J1, J2, J3. Through manipulation of the mobile terminal 20, the user A can provide answers to a plurality of inquiries included in the individual inquiry information to create individual evaluation information, and return the individual evaluation information within a given time period (at the times t3, t4, t5).

The plurality of inquiries are set to make it possible to estimate, from the individual evaluation information, at what level the user A has obtained the feeling of accomplishment, the feeling of self-fulfillment, the feeling of self-affirmation or the like, through each of the activities. Specifically, the inquiry may be set as follows: "Do you want to recommend this activity to others?", "Do you want to participate in this event again?", "Do you feel satisfied after experiencing this activity?", or "Did you help others in this activity?"

The individual evaluation information includes a subjective evaluation score of the satisfaction level, answered to each inquiry by the user. For example, the user A can choose one subjective evaluation score from a plurality of options. The options consist of, e.g., "Very good (100 points)", "Good (75 points)", "Not bad (50 points)", "Bad (25 points)" and "Very bad (0 point)". Alternatively, an evaluation method may be configured such that the user A chooses one evaluation score from between 100 points (Very good) to 0 point (Very bad).

When receiving the individual evaluation information from the user A, the management device 30 can use evaluation data included in the received individual evaluation information for the purpose of estimation of the WB value. For example, the WB value is estimated to be higher as the average of the evaluation scores for the plurality of inquiries is higher. The evaluation data indicates the level of a subjective evaluation made for each activity by the user A.

The management device 30 may be configured to transmit a voice analysis command to the mobile terminal 20, in conjunction with transmission of the individual inquiry information (e.g., at the time of transmission of the individual inquiry information, within a given time period from transmission of the individual inquiry information, at the time of receiving of the individual evaluation information, or within a given time period from receiving of the individual evaluation information). When receiving the voice analysis command, the mobile terminal 20 is operable to, after prompting the user A to vocally answer an inquiry, acquire voice data of the user, and analyze the acquired voice data to estimate the emotional state. The mobile terminal 20 returns analysis result data (emotion data indicating the estimated emotional state) to the management device 30. The management device 30 can use the analysis result data for the purpose of estimation of the WB value.

The user A can share an impression in each activity with a friend, using the communication function (particularly, SNS communication function) of the mobile terminal 20. Specifically, the user A can express an impression or a feeling of satisfaction, obtained in each activity, using text data or image data, and post this expression via the Internet line 3. Based on such a posting action, the WB value of the user A is maintained at a high level without attenuation. Further, in a similar manner to that mentioned above, the management device 30 is operable to receive, from the mobile terminal 20 (communication analysis device), the posting data analysis result data, and use the received analysis result data for the purpose of estimation of the WB value.

Even in a situation where, in the action period P2, the user A drives the vehicle 1 to move among the activities, the management device 30 is operable to receive analysis result data from the measurement device 10 of the vehicle 1 and the mobile terminal 20 (voice analysis device), and use the received analysis result data for the purpose of estimation of the WB value.

When the event E is terminated, the user A drives the vehicle 1 to return home (time t6), and returns to his/her daily life again. On the way home, the user A can immerse himself/herself in the feeling of self-fulfillment and the feeling of self-affirmation, while looking back on the recollection of the event E. The management device 30 is operable to receive analysis result data acquired by the measurement device 10 of the vehicle 1 and the mobile terminal 20 (voice analysis device) during driving, and use the received analysis result data for the purpose of estimation of the WB value.

In the fostering period P3, the recollection of the event E remains in the user A vividly, and the user A feels that the event E is still familiar. In the fostering period P3, the acquired positive emotions (feeling of self-fulfillment, feeling of self-affirmation) are organized in the user A, and a feeling of gratitude for people met in the event E is fostered. However, the WB value which has reached a high level by the participation in the event E gradually decreases over time in the fostering period P3.

In the fostering period P3, after the elapse of a first given time period (e.g., after several days to one week) from termination of the event E, the management device 30 transmits overall inquiry information to the mobile terminal 20 of the user A (time t7). This overall inquiry information is intended to check a satisfaction level of the user A with the overall event E. Through manipulation of the mobile terminal 20, the user A can provide answers to a plurality of inquiries included in the overall inquiry information to create overall evaluation information, and return the overall evaluation information (at the time 7), in a similar manner to that for the above-mentioned individual inquiry information.

The form of overall evaluation information is similar to that of the above-mentioned individual inquiry information. For example, the inquiry may be set as follows: "Do you want to recommend this activity to others?", "Do you want to participate in this event again?", "Do you feel satisfied after experiencing this activity?", or "Did you help others in this activity?" When receiving the overall evaluation information from the user A, the management device 30 can use evaluation data included in the received overall evaluation information for the purpose of estimation of the WB value. The evaluation data indicates the level of a subjective evaluation made for the overall event E by the user A. With regard to the overall inquiry information, the management device 30 may be configured to transmit a voice analysis command, in a similar manner to that for the above-mentioned individual inquiry information. In response to this command, the mobile terminal 20 is operable to execute voice analysis processing, and return resulting analysis result data to the management device 30.

In the fostering period P3, after the elapse of a second given time period (e.g., after one week to several weeks) from termination of the event E, the management device 30 transmits follow-up information to the mobile terminal 20 of the user A (time t8). The follow-up information is provided as a means to give the user A an opportunity to express gratitude to event-related persons. In response to the follow-up information, the user A can return gratitude expression information indicating gratitude. The event-related persons include all persons involved in the event E, and particularly include stuffs running the event E, volunteer staffs, and community people.

The user A can provide answers to a plurality of inquiries included in the follow-up information to create gratitude expression information. Examples of answer items include: items for identifying a target person to which gratitude is expressed (location, time, gender, keyword, etc.), and the level of gratitude ("High", "Medium", "Low", etc.). The answer items may include an item in which the user A can input a message, and an item for identifying the after-mentioned virtual currency transfer amount. Thus, the gratitude expression information includes a target person to which gratitude is expressed, and the level of gratitude to the target person.

This allows the user A to easily express gratitude to the target person in the fostering period P3 even in a situation where the user A does not know contact information of the target person (e.g., the user A hesitated and could not ask for the contact information), so that it is possible to promote organizing the positive emotions. Further, there is a possibility that the user A obtains a better feeling of self-fulfillment or a better feeling of self-affirmation. It should be understood that, when there is no target person to which gratitude is expressed, the user A needs not transmit the gratitude expression information.

Then, in the establishment period P4, after the elapse of a given time period (e.g., after two or three months) from termination of the event E, the management device 30 transmits memory establishment information to the mobile terminal 20 of the user A (time t9). This memory establishment information is intended to provide memory establishment promotion information (in this embodiment, photographic album) from the event provider B to the user A, so as to establish the recollection of the event E in the memory of the user A. The management device 30 is operable to capture photograph data taken by the event provider B during the event E and photograph data provided to the management device 30 by the event participants, and create a photographic album through automation edit of the photograph data using a photographic album creation application.

The management device 30 publishes created photographic album data on the website. The user A can access the photographic album on the website via the Internet line 3, through the use of access information included in the memory establishment information and by using the browser function of the mobile terminal 20. Alternatively, the management device 30 may be configured to transmit the created photographic album data to the mobile terminal 20, together with the memory establishment information.

Through browsing of the photographic album, the user A recalls the event E, and reconfirms the event E as a past event. In this way, in the establishment period P4, the event E is regarded as a past recollection in the user A, and the recollection of the event E is established in the memory of the user A. In the establishment period P4, the WB value also gradually decreases over time. However, the positive emotions obtained through the event E are accumulated unconsciously in the user, or in deep psychology of the user A.

During browsing of the photographic album, the user A can transmit confirmation information to the management device 30 by using the mobile terminal 20. When feeling that the participated event is satisfactory, the user A can return the confirmation information to the management device 30 by choosing a confirmation button on a page for browsing the photographic album.

When the confirmation information is returned, the user A can be deemed to feel that the participated event E is satisfactory. Thus, it is expected that the user A will hereinafter act as an "ambassador" who informs a friend or the like of a new event and encourages participation in the event. Alternatively, the confirmation information may be configured to be created and transmitted by allowing the user to provide answers to a plurality of inquiries similar to the inquiries of the overall inquiry information, in connection with the memory establishment information.

Next, data for estimating the WB value in this embodiment will be described. In this embodiment, for the purpose of estimation of the WB value, a plurality of types of data consisting of the following low-order to high-order data can be used.

Firstly, in order to estimate the WB value, the biological data (low-order data) provided from the measurement device 10 can be used. The biological data is obtained by a passive measurement of the user A, and is indicative of an objective physiological state of the user A. The biological data is measured continuously or intermittently during driving of the vehicle 1 by the user A. A measurement time period necessary to obtain significant information from the biological data is in the range of several seconds to several minutes. Further, the physiological state of the user A can change in several seconds to about one hour. Thus, a period during which meaningful or effective information can be obtained (effective period) is considered to be in the range of several minutes to several ten minutes including a period during the measurement or a duration of the measurement.

The measurement device 10 analyzes the mental state (i.e., tension level) of the user A, using the biological data, and outputs the analyzed mental state as analysis result data. Then, the management device 30 is operable to estimate the WB value at intervals of the measurement time period, using the received analysis result data, and store the estimated WB value (hereinafter referred to occasionally as "$WB_B$ estimate value") in the memory 30b. For example, the $WB_B$ estimate value is in the range of 0 (at which the WB value is smallest) to 100 (at which the WB value is largest). In one example, when the tension level is at an optimal medium level (tension level = 5), the $WB_B$ estimate value is estimated as a largest value ($WB_B$ estimate value = 100), and, as the tension level more largely deviates from the optimal value, the $WB_B$ estimate value is estimated as a smaller value. The analysis result information about the mental state obtained from the biological data is low-order information which does not directly correlate with the emotions (delight, anger, sorrow, pleasure, etc.) of the user A. Therefore, the $WB_B$ estimate value estimated from the biological data is low in terms of credibility, in some cases.

Secondly, in order to estimate the WB value, the analyses result data or the emotion data (intermediate data) provided by the voice analysis device can be used. The emotion data is obtained by analyzing a voice of the user A which is interactively produced between the voice analysis device and the user A, and is indicative of an objective emotional state of the user A. The emotional data is a result of accurately estimating the emotions of the user A. A measurement time period necessary to obtain the emotion data is about several seconds. Typically, the emotions of the user A can change in several minutes to about one hour. Therefore, the effective period for obtained information is considered to be about several ten minutes including a duration of the measurement.

The mobile terminal 20 outputs, as an analysis result data, the emotion data including the rate of each emotion and the mental soundness level, as mentioned above. Then, the management device 30 is operable to estimate the WB value at intervals of the measurement time period, using the received analysis result data, and store the estimated WB value (hereinafter referred to occasionally as "$WB_V$ estimate value") in the memory 30b. For example, the WBv estimate value is in the range of 0 to 100, as with the $WB_B$ estimate value. In one example, the management device 30 can employ the mental soundness kevel (0 to 100) as the $WB_V$ estimate value. Here, the $WB_B$ estimate value estimated from the voice analysis is an objective estimate value directly correlating with the emotions of the user A, so that it is generally high in terms of credibility. However, for example, in a long-distance walking event, although a great feeling of accomplishment can be obtained at a goal point, walking between course spots involves pain. Thus, in this type of event, there is a possibility that the $WB_V$ estimate value is estimated to be relatively small as a whole.

Thirdly, in order to estimate the WB value, the analysis result data (intermediate data) generated by the mobile terminal serving as the communication analysis device can be used. However, this analysis result data is obtained by analyzing subjective posting data of the user A, so that there is a case where the analysis result data is not exactly indicative of an objective emotional state of the user, (a case where the posting data is different from the intent of the user A, or the like). In this connection, when the user A posts a message or the like by using the SNS communication function, a friend or the user A himself/herself can transmit a further message with respect to this posting. Thus, in a case where one posting from the user A is analyzed together with any subsequent related posting, there is a possibility that the analysis needs to be continued over several minutes to several days. Therefore, the effective period for obtained information can extend over a period during which the user communicates with a friend.

The mobile terminal 20 outputs the emotional data as an analysis result data, through communication analysis processing, as mentioned above. Then, the management device 30 is operable to estimate the WB value at intervals of the analysis time period, using the received analysis result data, and store the estimated WB value (hereinafter referred to occasionally as "$WB_C$ estimate value") in the memory 30b. For example, the WBc estimate value is in the range of 0 to 100, as with the $WB_B$ estimate value. In one example, the management device 30 is operable to multiply a product of the rate of each of the four emotions and the strength of a corresponding one of the emotions by a coefficient set for a corresponding one of the emotions. The total value of the obtained products can be employed as the $WB_C$ estimate value.

Fourthly, in order to estimate the WB value, the subjective evaluation data made by the user A with respect to each activity or the event (high-order data) can be used. What is necessary to obtain the evaluation data is a progress in the implementation period of each activity or the event. The evaluation data is information about evaluation subjectively made by the user, so that it is lower than the data obtained using the measurement device 10 and the voice analysis device, in terms of objectivity. However, through analysis of the evaluation data, it is possible to estimate a high-order emotional state of the user A with respect to each activity or the event E.

The management device 30 is operable to receive the evaluation data from the mobile terminal 20 to estimates the WB value, based on a total value of the evaluation scores for respective inquiries, and store the estimated WB value (hereinafter referred to occasionally as "$WB_E$ estimate value") in the memory 30b. For example, a percentage of a total score p for all inquiries with respect to a perfect score (m points out of m for all inquiries) (p/m $\times$ 100) can be used as the $WB_E$ estimate value.

Fifthly, in order to estimate the WB value, the action log data (high-order data) can be used. The management device 30 is operable, based on the action log data of the user A, to analyze how many activities among various activities scheduled for the event E the user A has experienced, to estimate the feeling of accomplishment of the user A, and store the resulting estimate in the memory 30b. What is necessary to obtain the action log data is a progress in the implementation period of the event.

At the time of termination of the event E, the management device 30 determines, from the action log data, in how many activities among the plurality of activities prepared in the event E the user A has participated. For example, in a case where the user A has participated in m activities among n activities prepared in the event E, a participation rate (n/m $\times$ 100) can be used as the WB value (hereinafter referred to occasionally as "$WB_L$ estimate value"). In one example, in an event whose goal is to walk or run the entire course as in a long-distance walking event, it is effective to employ the method of estimating the $WB_L$ estimate value using the action log data.

Sixthly, in order to estimate the WB value, event participation record data obtained based on the action log data (high-order data) of the user A may be taken into account. Specifically, the event participation record data indicates data of the user A over past several years, including the number of times of participation in event, the frequency of participation in event, and the type of participated event. Based on analysis of such event participation record data, it is possible to estimate a higher-order (societal) emotional state of the user A with respect to an event participation activity, over several months to several years. Through repetitive participations in event, the feeling of self-fulfillment repeatedly arises in the user A, and will be accumulated unconsciously in the user A. Such repetitive experiences of the feeling of self-fulfillment over several years are considered to exert a good influence on the personality of the user A. The management device 30 is operable to acquire the event participation record data of the user A to calculate a cumulative WB value, e.g., by multiplying the number of times of participation in event by a coefficient, and store the calculated WB value (hereinafter referred to occasionally as "$WB_R$ estimate value") in the memory 30b.

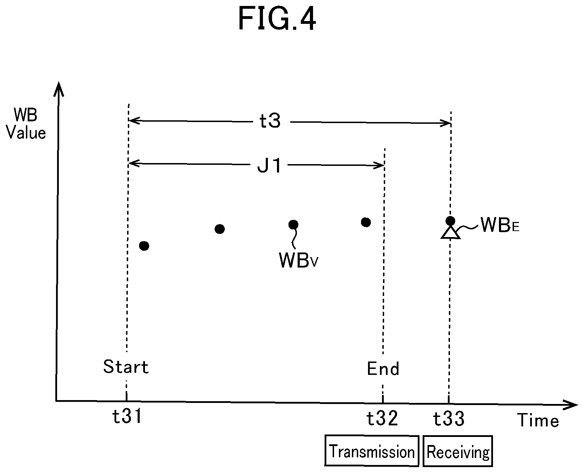

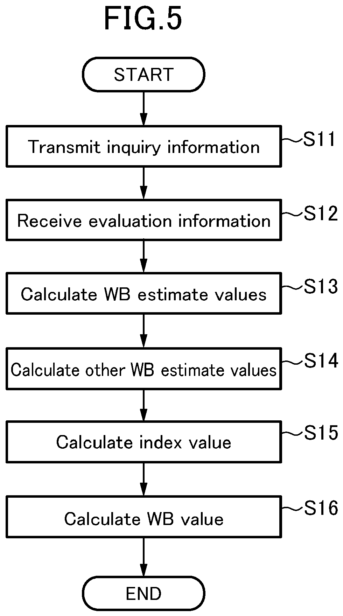

Next, with reference to FIGS. 4 and 5, a method of estimating the WB value in this embodiment will be described. FIG. 4 is an explanatory diagram of estimation of the WB value in an activity, and FIG. 5 is a processing flow for estimating the WB value in the activity. As mentioned above, the management device 30 is operable to estimate the WB value ($WB_B$ estimate value, $WB_V$ estimate value, $WB_C$ estimate value, $WB_E$ estimate value, $WB_L$ estimate value, $WB_R$ estimate value), using a single piece of data (only low-order data, only intermediate data, or only high-order data), in principle.

These WB estimate values vary in terms of time length of a target period, depending on data used. For example, the time length of the target period is: several second to several minutes for the $WB_B$ estimate value and the $WB_V$ estimate value; a period of each posting for the $WB_C$ estimate value; an implementation period of each evaluation target (i.e., an implementation period of each activity, or an implementation period of each event) for the $WB_E$ estimate value; and an implementation period of each event for the $WB_L$ estimate value; and several months to several years for the $WB_R$ estimate value. In one example, as shown in FIG. 4, with respect to the activity J1, the $WB_V$ estimate value can be estimated singly or plurally in the implementation period thereof, whereas the $WB_E$ estimate value is estimated only singly.

The management device 30 may be configured to combine the plurality of types of data different in the time length of the target period so as to estimate a WB value more conforming to an actual situation. Specifically, by assuring the credibility of relatively high-order data based on combining with relatively low-order data, or conversely by assuring the credibility of relatively low-order data based on combining with relatively high-order data, it becomes possible to use assured data for the purpose of estimation of the WB value.

More specifically, the $WB_B$ estimate value, the $WB_C$ estimate value, the $WB_E$ estimate value, the $WB_L$ estimate value and the $WB_R$ estimate value, except for the $WB_v$ estimate value, do not exactly reflect the objective emotion data of the user A. Thus, in this embodiment, the management device 30 is configured to, during estimation of the WB value, correct the $WB_B$ estimate value, the $WB_C$ estimate value, the $WB_E$ estimate value, the $WB_L$ estimate value or the $WB_R$

estimate value, by the $WB_V$ estimate value as an emotion index value. For example, the WB value can be calculated by the following formula.

$$\text{WB value} = (\text{coefficient } kv \times WB_V \text{ estimate value}) + \sum (\text{coefficient } kn \times WBn$$
$$\text{estimate value} \times WB_V \text{ estimate value}) + \sum (\text{adjustment value } Mn) \text{ (where } n = B, C, E, L,$$
$$R)$$

That is, the WB value is calculated by summing: a value obtained by multiplying the $WB_V$ estimate value by the coefficient kv; a value obtained by multiplying a product of each value of the WBn estimate value and the WBv estimate value by a corresponding value of the coefficient kn; and the adjustment value Mn corresponding to each value of the WBn estimate value. It should be noted that the estimate values other than the $WB_V$ estimate value need not necessarily be used all. Thus, each value of the coefficients kv, kn and the adjustment value Mn can be set differently depending on a combination of the different types of data used.

In this embodiment, in a case where a plurality of types of data are used, the plurality of types of data are obtained during mutually corresponding time periods, respectively. For example, in a case where the WB value is estimated for the activity J1 of the event E, a plurality of types of data measured during the implementation (target) period (which is approximately equal to a time period t3) of the activity J1 are used. On the other hand, in a case where the WB value is estimated for the overall event E, a plurality of types of data measured during the implementation period (a time period between t2 to t6) of the event E are used in principle. It should be noted here that an average value during the implementation period or a value at a specific time point in the implementation period may be employed as the data used.

A method of estimating the WB value for the activity J1 of the event E will be specifically described below. The holding (implementation) period of the activity J1 is set in the event data. As shown in FIG. 4, a start time 31, an end time t32, and an inquiry information collection time (t32 + a given time period $\Delta t$) are set in the event data. Based on the read event data, the management device 30 transmits the individual inquiry information to the mobile terminal 20 at the end time t32 (step S11 in FIG. 5), and, at time t33 before the elapse of the given time period $\Delta t$ from the transmission, receives the individual evaluation information (step S12 in FIG. 5).

Based on the evaluation data in the individual evaluation information received at the time t33, the management device 30 calculates the $WB_E$ estimate value for the activity J1 (See $\Delta$ in FIG. 4; step S13 in FIG. 5). Further, the management device 30 intermittently receives the voice analysis-based analysis result data from the mobile terminal 20 to calculate the WBv estimate value at the time of each receiving of the analysis result data, and stores the calculated $WB_V$ estimate value in the memory 30b (see • in FIG. 4).

In a case where the measurement device 10 is configured as a mobile biological information measurement device, the management device 30 calculates the average of $WB_B$ estimate values, or the $WB_B$ estimate value around the time t33, based on the mental state analysis result data received during the target period (step S14 in FIG. 5). Further, the management device 30 calculates the WBc estimate value based on the communication analysis-based analysis result data received during the target period (step S14 in FIG. 5).

The management device 30 calculates the emotion index value, using WBv estimate value calculated correspondingly to the target period (t31 to t33) of the activity J1 (step S15 in FIG. 5). In this embodiment, as shown in FIG. 4, the activity J1 is an activity of a type in which the $WB_V$ estimate value is kept approximately at a constant value over the period, wherein the $WB_V$ estimate value is approximately coincident with the $WB_E$ estimate value. For example, the management device 30 averages out a plurality of $WB_V$ estimate values corresponding to the target period, and sets the resulting average value as the emotion index value. Alternatively, the $WB_V$ estimate value at a specific time point (e.g., at the time t32, t33 or the like), or an average of WBv estimate values in a specific period (e.g., period between t32 to t33), may be used as the emotion index value.

Based on the $WB_V$ estimate value, the $WB_E$ estimate value and others, the management device 30 calculates the WB value for the activity J1, as mentioned above (step S16 in FIG. 5). More specifically, the WB value is calculated, using the calculated emotion index value, the $WB_E$ estimate value and others.

In a method of estimating the WB value for the overall event E, the management device 30 executes similar processing to the professing steps in FIG. 5, using a variety of data measured in the target period (t2 to t7) of the event E. Specifically, the $WB_E$ estimate value is calculated from the evaluation data in the overall evaluation information obtained at the time t7 (see the step S13).

Further, other WB estimate values are calculated from the mental state analysis result data based on the biological data, the communication analysis-based analysis data, and the action log data in the event E, obtained during the period between the times t2 to t6 (if necessary, t2 to t7) (see the step S14). Further, the index value is calculated from the voice analysis-based analysis result data obtained during the period between the times t2 to t6 (if necessary, t2 to t7) (see the step S15). Then, the management device 30 calculates the WB value using the calculated values (see the step S16).

In this embodiment, the measurement device 10 and the mobile terminal 20 process the measurement data, and transmit the processed data (analysis result data, emotion data, etc.) to the management device 30, and the management device 30 estimates the WB value based on the processed data. Alternatively, the management device 30 may be configured to receive the measurement data from the measurement device 10 and the mobile terminal 20, and estimate the WB value based on the received measurement data.

Next, with reference to FIG. 3B, virtual currency obtained by the user A in connection with the event E will be described. FIG. 3B is a graph showing a temporal change in the balance of virtual currency of the user. The LA system according to this embodiment is configured such that virtual currency is given to the user A in a given amount (unit is denoted as "P") according to the WB value to be estimated, in a manner triggered by a given manipulation of the user A.

The virtual currency in this embodiment is set to have two types of attributes (attenuation type and invariable type). That is, the attribute of the virtual currency can be changed between attenuation-type virtual currency and invariable-type virtual currency. The attenuation-type virtual currency has a time attenuation characteristic in which the amount thereof itself decreases over time (e.g., decreases by half in one week). The attenuation rate may be a linear or stepwise rate, or may be an exponential rate. On the other hand, the invariable-type virtual currency is a non-attenuation-type virtual currency having no time attenuation characteristic.

The following description will be made on the assumption that the virtual currency when being given to the user A is the attenuation type. The attenuation-type virtual currency can be changed to the invariable-type virtual currency at a given timing.

In the preparation period P1, when the user A transmits the entry information from the mobile terminal 20 to the management device 30 (time t1), the management device 30 gives a given amount (e.g., 20 P) of virtual currency to the user A, in response to receiving of the entry information. That is, it is assumed that, when transmitting the entry information, the user A comes into a state in which the WB value rises beyond a normal value. Thus, the management device 30 adds a given amount to the virtual currency data in the user data. This given amount may be a fixed value, or may be a value according to an entry fee to be payed to the event provider B.

In the action period P2, with regard to each of the activities J1 to J3, the management device 30 estimate the WB value (at each of the times t3, t4, t5), based on the individual evaluation information, and the voice analysis-based analysis result data, and gives virtual currency to the user A in a given amount according to the estimated WB value. For example, when the estimated WB value is greater than a given value, a given amount (fixed amount) of virtual currency is given to the user A. In FIG. 3B, correspondingly to each of the activities J1, J2, J3, virtual currency is given to the user A in a given amount (e.g., 40 P, 50 P or 30 P).

Note that the virtual currency given in the preparation period P1 and the action period P2 is the attenuation type. Thus, attenuation is started from a time point at which the virtual currency is given, or after the elapse of a given non-attenuation period (e.g., several days to several weeks) from the time point at which the virtual currency is given, at a given attenuation rate.

In the fostering period P3, with regard to the event E, the management device 30 estimate the WB value, based on the overall evaluation information received from the user A (and, when needed, the analysis result data about user's voice during returning of the overall evaluation information, and a variety of analysis result data during the event period). The management device 30 may be configured to set the attenuation rate of virtual currency in the user data, based on the estimated WB value. Specifically, the attenuation rate may be set to be lower as the estimated WB value becomes increasingly greater than a given value.

In the fostering period P3, when the user A returns, in response to the follow-up information, the gratitude expression information by using the mobile terminal 20 (time t8), a given transfer amount F of virtual currency is transferred from the user A to the event provider B. Specifically, the management device 30 changes the attribute in the virtual currency data stored in the user data of the user A, thereby converting the transfer amount F of attenuation-type virtual currency to the same amount of invariable-type virtual currency. Then, the management device 30 subtracts the transfer amount F of invariable-type virtual currency from the user data of the user A, and adds the transfer amount F of invariable-type virtual currency to the virtual currency data in the user data of the event provider B.

Here, the event provider B receives the transfer amount F of invariable-type virtual currency on behalf of the event-related persons. The event provider B can transmit a gratitude message received from the user A, to one or more persons specified in a reply to the follow-up information, together with the transfer amount F of invariable-type virtual currency. In this way, through involvement in the operation of the event E, the event-related persons can receiver gratitude, and obtain an economic value. This becomes an incentive for the event-related persons to further improve the event E from a viewpoint of WB.

The transfer amount F may be a prescribed value, or may be a value selected by the user A in one answer item regarding the transfer amount, included in the follow-up information. For example, in the case where the transfer amount F is a prescribed value, the prescribed value may be set according to the WB value estimated based on the answer to the overall inquiry information (as the estimated WB value is larger, the prescribed value is set to a larger value), or the prescribed value may be a given rate (e.g., 30%) of the virtual currency obtained by the user A in connection with the event E. Further, when the transfer amount F of virtual currency is transferred to the event provider B, it needs not necessarily be subtracted from the virtual currency data of the user A.

In the fostering period P3, when the user A returns, in response to the memory establishment information, the confirmation information by using the mobile terminal 20 (time t8), the management device 30 converts a given amount G of attenuation-type virtual currency in the virtual currency data of the user A, to the same amount of invariable-type virtual currency. In this embodiment, the management device 30 converts a given percentage (0 to 100%) of the attenuation-type virtual currency obtained in the event E and existing in the virtual currency data of the user A at the time of receiving of the confirmation information, to invariable-type virtual currency. This given amount G of invariable-type virtual currency is returns for the user A undertaking the "ambassador".

The given percentage may be a fixed value (e.g., 50%), or may be a variable value. For example, in the case where the given percentage is a variable value, the variable value may be set according to the magnitude of the WB value estimated based on the overall evaluation information. In one example, the variable value is set to be larger as the estimated WB value is larger.

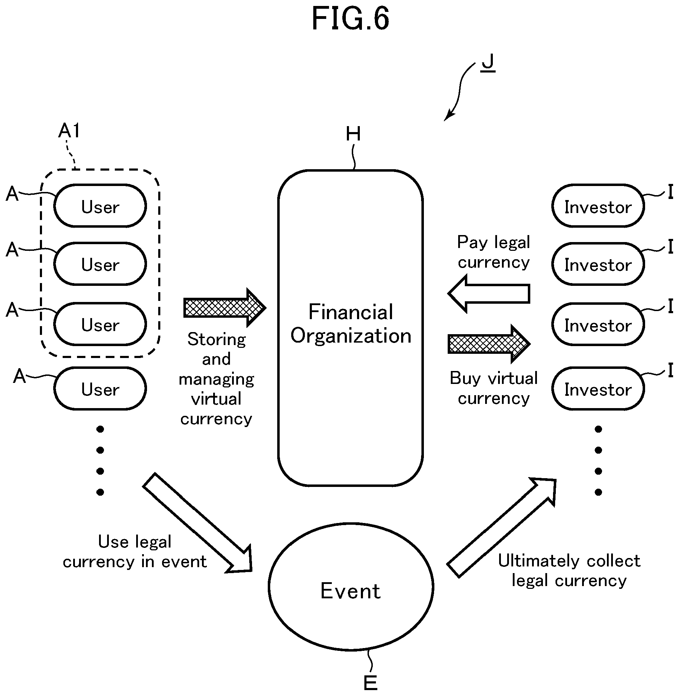

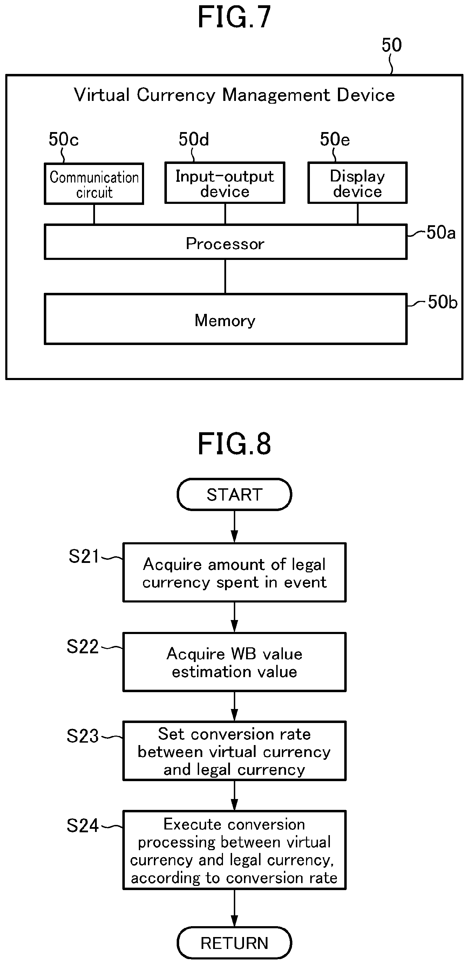

Next, with reference to FIGS. 6 and 7, a transaction system using the above-mentioned virtual currency will be described. FIG. 6 is a conceptual diagram of a virtual currency transaction system according to another, second, embodiment of the present invention, and FIG. 7 is a schematic configuration diagram of a virtual currency management device in the second embodiment.

As shown in FIG. 6, the virtual currency transaction system J mainly comprises: a plurality of users A each serving as a subject who participates in the event E and generates virtual currency; a financial organization H managing the virtual currency generated by each user A; and a plurality of investors I as a third party selling/buying virtual currency to/from the financial organization H.

The financial organization H is typically equivalent to a trust bank engaged in trust business. Specifically, the financial organization H is realized by the virtual currency management device 50 as shown in FIG. 7. The virtual currency management device 50 is composed of a computer comprising a processor 50a, a memory 50b, a communication circuit 50c, an input-output device 50d, and a display device 50e, and configured to cause the processor 50a to execute various programs stored in the memory 50b, thereby performing various processings.

The communication circuit 50c of the virtual currency management device 50 is operable to transmit and receive a variety of data between terminal devices each of which has at least communication function and is used by each user A or investor I. In this case, the virtual currency management device 50 is provided with a personal information blocking wall to prevent data about users A and the like from leaking to the outside. The input-output device 50d comprises a key board or touch sensor-type input device, a microphone, and a speaker, and the display device 50e is composed of, e.g., a liquid crystal display unit having a touch panel function. The input-output device 50d and the display device 50e are used by each user A, each investor I, the event provider B and others.

In addition to programs to be executed by the processor 50a, a variety of information is stored in the memory 50b of the virtual currency management device 50. Specifically, the amount of virtual currency generated by each user is stored in the memory 50b in association with the user. Particularly, this amount of the virtual currency is stored in a state in which it is further associated with information about the amount of legal currency spent by the user in the event E in which the virtual currency is generated, information about the accuracy of estimation of the WB value as the basis for generating the virtual currency. Further, information about a given group to which each user belongs (see the broken line A1 in FIG. 6; this group will hereinafter be referred to appropriately as "user group A1") is stored in the memory 50b. Specifically, as the information about the user group A1, the type of group (e.g., sports circle, lesson circle, car sharing club, local activity group, etc.), users belonging to the group, the rank of each user in the group (member rank, etc.), etc., are stored in the memory 50b. It should be noted here that, in addition to groups such as a sports circle, a lesson circle, a car sharing club, and a local activity group, the user group A1 also includes a subgroup obtained by dividing such a group by rank in the group (e.g,. each rank group such as silver members, or gold members).

This virtual currency management device 50 may be realized by the aforementioned management device 30 (FIG. 2) of the event provider B. That is, instead of providing the virtual currency management device 50 separately from the management device 30, the management device 30 may be configured to additionally function as the virtual currency management device 50. In this case, the event provider B (i.e., an operating company of the event E) may fulfill the function of the financial organization H. In this case, the virtual currency management device 50, i.e., the management device 30, is equivalent to "virtual currency management device" set forth in the appended claims. The following description will be made by taking, as an example, a configuration in which the management device 30 functions as the virtual currency management device 50, i.e., the virtual currency management device 50 is identical to the management device 30.

It should be understood that, in another embodiment, the virtual currency management device 50 may be provided separately from the management device 30. In this case, the event provider B (i.e., an operating company of the event E) needs not fulfill the function of the financial organization H. In this case, each of the virtual currency management device 50 and the management device 30 is equivalent to "virtual currency management device" set forth in the appended claims.

Here, the operation (flow of a virtual currency transaction, etc.) of the virtual currency transaction system J according to the second embodiment illustrated in FIG. 6 will be specifically described.

First of all, as mentioned above, when a user A participates in an event E provided by the event provider B, the virtual currency management device 50 as the financial organization H estimates the WB value in a period during which the user A participates in the event E, and generates virtual currency in an amount according to the estimated WB value. Then, the virtual currency management device 50 stores the generated virtual currency in the memory 50b in association with the user A, and manages the generated virtual currency in association with the user A. When the user A participates in a plurality of events E, the virtual currency management device 50 stores a cumulative amount of virtual currency generated in the events E. In this case, it is preferable to store individual amounts of virtual currency generated in the events E, in association with respective ones of the events E, together with the cumulative amount of the virtual currency.

Further, the virtual currency management device 50 acquires the amount of legal currency spent in the event E by the user A, and stores the acquired amount of the legal currency in a state in which it is further associated with the virtual currency. For example, the virtual currency management device 50 is operable to receive, from the event provider B or the cooperator C of the event E (see FIG. 1), provision of information about the amount of legal currency spent in the event E by the user A (in this case, it is desirable to obtain, from the user A, approval of provision of the information in advance). Further, in the case where the user A participates a plurality of events E, the virtual currency management device 50 determines a cumulative amount or average amount of respective amounts of legal currency spent in the plurality of events E by the user A, and stores the cumulative amount or average amount of the amounts of the legal currency.

Further, the virtual currency management device 50 stores the accuracy (i.e., credibility, which can be basically indicated by a numerical value) of estimation of the WB value determined in the event E in which the user A participates. In the case where the user A participates a plurality of events E, the virtual currency management device 50 determines an average estimation accuracy with regard to the WB values estimated in the plurality of events E, and stores this average estimation accuracy.

As mentioned above, the WB value is determined based on at least one of the following six evaluate values: the $WB_B$ estimate value based on biological data; the WBv estimate value based on voice analysis; the $WB_C$ estimate value based on communication analysis; the $WB_E$ estimate value based on subjective evaluation data; the $WB_L$ estimate value based on action log data; and the $WB_R$ estimate value based on event participation record data. In one example, the virtual currency management device 50 sets the WB value estimation accuracy (numerical value), according to the number of evaluate values actually used for determining the WB value, among the six evaluate values. Specifically, as the number of evaluate values actually used for determining the WB value, among the six evaluate values, becomes larger, the virtual currency management device 50 sets the WB value estimation accuracy to a higher value. For example, the virtual currency management device 50 is operable to specifically set the value of the evaluation accuracy, in such a manner that: when all of the six evaluate values are used, the estimation accuracy is set to 100; when only five evaluate values are used, the estimation accuracy is set to 80; when only four evaluate values are used, the estimation accuracy is set to 60; when only three evaluate values are used.

In another example, the virtual currency management device 50 sets a rank regarding the level of the estimation accuracy, for each of the six estimate values, and determines the estimation accuracy according to the rank of an actually used estimate value among the six estimate values. Specifically, the virtual currency management device 50 sets a score of each rank regarding the estimation accuracy, and determines, as the estimation accuracy, the total of scores corresponding to respective ranks of one or more actually-used estimate values among the six estimate values. For example, the $WB_E$ estimate value based on subjective evaluation data is preferably set to a higher rank (in one example, to a higher rank than the $WB_B$ estimate value based on biological data, or the $WB_V$ estimate value based on voice analysis).

The legal currency amount and the WB value estimation accuracy to be stored in association with the virtual currency in the above manner substantially indicate a characteristic regarding the WB value of the user A himself/herself who generated the virtual currency, specifically the easiness for the user A to become the well-being state (i.e., the strength or magnitude of the tendency to become the user A to become the well-being state) (this characteristic will hereinafter be referred to as "WB characteristic"). That is, a user A who spends a relatively large amount of legal currency in an event E is relatively large can be said to exhibit a strong WB characteristic, i.e., have a strong tendency to become the well-being state. Further, a user A whose WB value estimation accuracy is relatively high is considered to willingly perform measurements, a response to each inquiry, posting through SNS, etc., for estimating the WB value in an event (i.e., to be cooperative in estimating the well-being value), and can be said to exhibit a strong WB characteristic. Virtual currency generated by such a user A who exhibits a strong WB characteristic can be said to be virtual currency having high credibility (monetary value) with respect to investment by the investor I. The details of this will be described later.

Then, the virtual currency management device 50 sells/buys the virtual currency generated and stored in the above manner, to/from each inventor I as a third party (since each investor I basically does not participate in the event E, the term "third party" is used for the investor(s) I from a standpoint of distinguishing the investors I from the users A participating in the event E). In this case, the virtual currency management device 50 performs processing for selling the virtual currency being managed, to an investor I, and performs processing for buying out the virtual currency bought by this investor I. Specifically, when selling virtual currency, the virtual currency management device 50 perform processing of converting legal currency payed by an investor I to virtual currency by applying a given conversion rate to the legal currency, and giving the converted virtual currency to the investor I. In this case, the virtual currency management device 50 stores the virtual currency given to the investor I in the memory 50b in association with the investor I. On the other hand, when buying out virtual currency, the virtual currency management device 50 perform processing of converting virtual currency payed by an investor I to legal currency by applying a given conversion rate to the virtual currency, and giving the converted legal currency to the investor I. For example, the virtual currency management device 50 performs processing of transferring the legal currency given to the investor I, to an account of the investor I. The conversion rate between virtual currency and legal currency is variable, and the virtual currency management device 50 also performs processing of setting the conversion rate.

Further, the virtual currency management device 50 sells/buys virtual currency to/from each inventor I, in units of virtual currency owned by a single user A, or in units of virtual currency owned by a user group A1 comprising a plurality of users A (this virtual currency means the sum of respective virtual currency sets each owned by a respective one of the plurality of users A; the same is applied to the following). Thus, the investor I can buy virtual currency owned by a specific user A, or virtual currency owned by a specific user group A1, instead of widely-circulated virtual currencies. When the investor I buys the virtual currency of the specific user A or the specific user group A1, the virtual currency management device 50 manages the specific user A or the specific user group A1 by assigning a flag or the like thereto, so as to allow a given benefit to be given to the specific user A or the specific user group A1. For example, the virtual currency management device 50 sets a user A or a user group A1 from which virtual currency is bought by an investor I, as a prime member in the event E, so as to allow a benefit of the prime member to be given to the user A or the user group A1 in the event E. In one example, the user A or the user group A1 who became the prime member can get a discount for an event entry fee, a discount voucher usable in an event venue, or commemorative goods relevant to the event E.

In addition, the virtual currency management device 50 sets a conversion rate between virtual currency and legal currency, in units of virtual currency owned by a single user A, or in units of virtual currency owned by a user group A1. Specifically, the virtual currency management device 50 sets the conversion rate to cause a monetary value of virtual currency with respect to legal currency to be increased as the aforementioned credibility (monetary value) of the virtual currency is higher (setting the conversion rate in this manner will hereinafter be expressed simply as "raising the virtual currency conversion rate"). That is, the virtual currency management device 50 raises the virtual currency conversion rate as the amount of legal currency spent in the event E is larger, and raises the virtual currency conversion rate as the estimation accuracy of the WB value as the basis for generating the virtual currency is higher. In this case, virtual currency having high credibility is basically less risky in terms of investment and is highly popular, so that the investor I needs to pay a relatively large amount of legal currency at the time of buying of the virtual currency. On the other hand, with regard to virtual currency having high credibility, the investor I can obtain a relatively large amount of legal currency at the time of selling of the virtual currency. It should be noted here that the present invention is not limited to applying the conversion rate varying according to the credibility, both at the time of buying of virtual currency and at the time of selling of virtual currency as in the above embodiment. For example, a fixed conversion rate may be applied at the time of buying of virtual currency, and the conversion rate varying according to the credibility may be applied only at the time of selling of virtual currency.

Further, with respect to virtual currency having a higher conversion rate, the virtual currency management device 50 sets an upper limit of a legal currency amount by which each investor I is permitted to invest at the time of buying of virtual currency (investment upper limit amount) to a higher value. For example, the virtual currency management device 50 sets the investment upper limit amount to a higher value as the conversion rate becomes higher. This makes it possible to ensure a long-term investment (financial support) by investors I.

Further, the virtual currency management device 50 provides information about virtual currency to allow each investor I to know the virtual currency conversion rate in units of user A or user group A1, when buying virtual currency (e.g., the information is displayed on the display device 50e or the like). Thus, it is possible for the investor I to determine a user A or user group A1 having virtual currency to invest in, based on credibility of virtual currency corresponding to the virtual currency conversion rate. For example, the investor I can selectively invest in virtual currency of a user A or user group A1 having high credibility. Here, the virtual currency management device 50 may be configured to, when each investor I buys virtual currency, provide the virtual currency information to allow each investor I to further know in what kind of event E (including an area in which an event E is held) the user A or user group A1 participated, i.e., to know a record of participated events E. Thus, the investor I can selectively invest in virtual currency of a user A or user group A1 having a high frequency of participation in a specific type of event E or in an event E held in a specific area.

When the investor I buys virtual currency in units of user A or user group A1, it is preferable that, even after buying of the virtual currency, the virtual currency management device 50 continuously manages the bought virtual currency as virtual currency owned by the user A or user group A1 without vanishing virtual currency owned by the user A or user group A1 (i.e., without transferring the ownership of the virtual currency to the investor I. Thus, after buying of the virtual currency by the investor I, i.e., after investment in the virtual currency, the user A or user group A1 invested in can continue to participate in an event E using its owned virtual currency. However, the virtual currency management device 50 is preferably configured to, once the investor I buys the virtual currency, restrict buying of the bought virtual currency by another investor I, although the ownership of the user A or user group A1 with regard to the bought virtual currency is not vanished.

Meanwhile, the virtual currency management device 50 is preferably configured to basically prohibit the investor I from spending the virtual currency bought by the investor I (e.g., restrict participation in an event E, etc., using the bought virtual currency). However, the virtual currency management device 50 permits the investor I to sell the bought virtual currency, i.e., convert the bought virtual currency to legal currency. In this case, the virtual currency management device 50 is preferably configured to restrict selling of the bought virtual currency by the investor I, until a certain time period (e.g., 5 or 10 years) elapses since the investor I bought the virtual currency. This makes it possible to ensure a long-term investment (financial support) by investors I.

In another embodiment, when an investor I buys virtual currency in units of user A or user group A1, the virtual currency of the user A or user group A1 may be vanished. In this case, the investor I may be permitted to spend the virtual currency bought by the investor I, and legal currency corresponding to the bought virtual currency may be given to the user A or user group A1.

Here, with reference to FIG. 8, currency conversion processing in the second embodiment will be described. FIG. 8 is a processing flow for performing conversion between virtual currency and legal currency in the second embodiment. This flow is started by the virtual currency management device 50 (specifically, the processor 50a) when receiving a request for conversion between virtual currency and legal currency (specifically, a request for buying virtual currency or a request for selling virtual currency) from an investor I.

First of all, at step S21, with regard to target virtual currency which is subject to the request for conversion between virtual currency and legal currency, the virtual currency management device 50 acquires the amount of legal currency spent in an event E by a user A or user group A1 who generated the target virtual currency. Specifically, the virtual currency management device 50 reads the legal currency amount stored in association with the target virtual currency, from the memory 50b. In a case where the target virtual currency was generated in a plurality of events E, the virtual currency management device 50 acquired a cumulative amount or average amount of legal currency spent in the plurality of events E.

Then, at step S22, with regard to the target virtual currency which is subject to the request for conversion between virtual currency and legal currency, the virtual currency management device 50 acquires the WB value estimation accuracy as the basis for generating the target virtual currency. Specifically, the virtual currency management device 50 reads the WB value estimation accuracy stored in association with the target virtual currency, from the memory 50b. In the case where the target virtual currency was generated in a plurality of events E, the virtual currency management device 50 acquires an average estimation accuracy with regard to the WB values estimation accuracy in the plurality of events E.

Then, at step S23, the virtual currency management device 50 sets the conversion rate between virtual currency and legal currency, based on the legal currency amount and the WB values estimation accuracy acquired in the step S21 and the step S22, respectively. Specifically, the virtual currency management device 50 increases the virtual currency conversion rate as the legal currency amount is larger, and increases the virtual currency conversion rate as the WB values estimation accuracy is higher. For example, a map in which the conversion rate to be applied is associated with various values of the legal currency amount and various values of the WB values estimation accuracy is preliminarily created, and the virtual currency management device 50 refers to the map to set a value of the conversion rate corresponding to respective values of the legal currency amount and the WB values estimation accuracy acquired in the step S21 and the step S22, respectively.

Then, at step S24, the virtual currency management device 50 executes professing for the conversion rate between virtual currency and legal currency, using the conversion rate set at step S23. Specifically, when receiving the request for buying virtual currency, from an investor I, the virtual currency management device 50 applies the set conversion rate to legal currency paid by the investor I to convert the legal currency to virtual currency. On the other hand, when receiving the request for selling the virtual currency, from the investor I, the virtual currency management device 50 applies the set conversion rate to the virtual currency paid by the investor I to convert the virtual currency to legal currency. Then, one cycle of the processing flow in FIG. 8 is completed.

Returning to FIG. 6, the operation of the virtual currency transaction system J according to the second embodiment will be described. As shown in FIG. 6, in the virtual currency transaction system J, the financial organization H (which is equivalent to the virtual currency management device 50 (the management device 30) as mentioned above) also functioning as the event provider B provides an event E to each user A, and the user spends legal currency (i.e., performs a consumption behavior) during participation in the event E, and generates virtual currency according to the WB value. Each investor I can buy the virtual currency generated in this manner, from the financial organization H.

When an investor I buys the virtual currency, a given benefit in the event E is given to a user A or user group corresponding to the virtual currency. For example, a user A or user group whose virtual currency is bought by the investor I is set as a prime member in the participated event E, and can receive a benefit of the prime member. This encourages the user A or user group to be motivated to participate in the event E, and encourages a consumption behavior in the event E, i.e., it is expected to increase the legal currency amount to be spent in the event E.

Further, when the investor I buys the virtual currency, the financial organization H obtains legal currency corresponding to the virtual currency, from the investor I. The financial organization H as the event provider B uses the legal currency obtained from the investor I in the above manner, as organizing costs, operating costs, etc., of the event E. Further, the financial organization H can earn revenue from the event E. This revenue includes legal currency spent in the event E by users A, and funds provided by sponsors of the event E.

Meanwhile, when the investor I sells the virtual currency after the elapse of a given time period (typically, several years) from buying of the virtual currency, the financial organization H pays legal currency corresponding to the virtual currency, to the investor I. In a situation where the virtual currency conversion rate is higher at the time of selling of the virtual currency that at the time of buying of the virtual currency, the financial organization H pays legal currency in an amount greater than the legal currency payed at the time of buying of the virtual currency. In this case, the financial organization H allots the revenue earned in the event E and others as mentioned above, for legal currency to be payed at the time of selling of the virtual currency. Further, in this case, the investor I reaps profits from buying and selling of the virtual currency. In this way, the investor I can collect legal currency.

Here, an investor I basically tends to buy virtual currency having high credibility (monetary value), i.e., virtual currency having a high virtual currency conversion rate. Therefore, through buying of such virtual currency, the investor I invests in a user A or group user A1 who exhibits a strong WB characteristic, i.e., has a strong tendency to become the well-being state. In response to buying of the victual currency, a given benefit is given to the user A or user group A1, so that it is possible to further encourages the motivation to participate in the event E and further encourages the consumption behavior in the event E, i.e., it is expected to further increase the legal currency amount to be spent in the event E. Particularly, such a user A or user group A1 exhibiting a strong WB characteristic takes a variety of strong consumption behavior, and can expand a consumption behavior while involve people therearound. Therefore, it is possible to activate the event E, and further activate an area in which the event E is held. In this case, the invent provider B can effectively increase the revenue in the event E.

As above, through buying of the virtual currency (investment) by an investor I, it is possible to encourage a user A or user group A1 to participate in an event E and encourage a consumption behavior in the event E, thereby realizing activation of the event E and activation of area in which the event E is held. Here, in a case where it is intended to achieve activation of a specific type of event E or a specific area, directly through buying of the virtual currency, the investor I may selectively buy virtual currency of a user A or user group A1 having a high frequency of participation in the specific type of event E or in an event E held in the specific area.

Meanwhile, along with expansion of the consumption behavior by the user A or user group A1 exhibiting a strong WB characteristic as mentioned above, i.e., an increase in the amount of legal currency spent in the event E, credibility (monetary value) of the virtual currency generated by the user A or user group A1 is gradually raised, and the virtual currency conversion rate is gradually increased (the virtual currency conversion rate becomes higher than that at the time of buying of the virtual currency). Therefore, the investor I can reap a relatively large profit at the time of selling of the virtual currency. That is, since virtual currency of the user A or user group A1 exhibiting a strong WB characteristic has a high virtual currency conversion rate, the investor I needs to pay a relatively large amount of legal currency at the time of buying of the virtual currency. However, the virtual currency conversion rate of such virtual currency is highly likely to become higher than the initial value. Thus, the investor I can reap a relatively large profit at the time of selling of the virtual currency.

The above description has been made based on an example where an investor I invests in virtual currency of a user A or user group A1 exhibiting a strong WB characteristic, i.e., in virtual currency having a high virtual currency conversion rate. However, there is also a case where an investor I daringly invests in virtual currency of a user A or user group A1 exhibiting a low WB characteristic, i.e., in virtual currency having a low virtual currency conversion rate. In this case, virtual currency having a low virtual currency conversion rate (i.e., virtual currency having low credibility) is basically highly risky in terms of investment and is less popular, so that the investor I needs not pay a large amount of legal currency at the time of buying of the virtual currency. That is, the investor I can buy the virtual currency by a relatively small amount of legal currency. Meanwhile, in the user A or user group A1 whose virtual currency was bought by the investor I, even when the WB characteristic thereof is low at the time of buying of the virtual currency, participation in an event E and consumption behavior in the event E are likely to be encouraged by a benefit given at the time of buying of the virtual currency. Therefore, credibility of virtual currency subsequently generated tends to be gradually raised, and the virtual currency conversion rate tends to be increased (the virtual currency conversion rate tends to become higher than that at the time of buying of the virtual currency). Thus, even in the case where the investor I buys the virtual currency of the user A or user group A1 exhibiting a low WB characteristic, the investor I can reap a relatively large profit at the time of selling of the virtual currency.

Next, the effect of the virtual currency management device 50 according to the second embodiment of the present invention will be described.

In this embodiment, the virtual currency management device 50 is operable to estimate the WB value of a user who participates in an event E to generate virtual currency based on the estimated WB value, and sells/buys the virtual currency to/from a third party (investor I). This allows the third party to adequately invest in the virtual currency generated by the user A who participates in the event E. Therefore, it becomes possible to encourage the user A or the like to participate in the event E, and encourage a consumption behavior in the event, through buying of the virtual currency (investment) by the third party, and thereby realize activation of the event E, activation of an area in which the event is held, etc.

In this embodiment, the virtual currency management device 50 is operable to sell/buy virtual currency to/from the third party, in units of virtual currency of a single user A or in units of virtual currency of a given user group A1. This allows the third party to limitedly invest in virtual currency of a specific user A or virtual currency of a specific user group A1.

In this embodiment, the virtual currency management device 50 is operable to set the conversion rate between virtual currency and legal currency, with respect to each virtual currency of a single user A, or with respect to each virtual currency of a given user group A1, so that it becomes possible to adequately set the virtual currency conversion rate, according to credibility (monetary value) of the virtual currency of each single user A or each user group A1.

In this embodiment, the virtual currency management device 50 is operable to increase the virtual currency conversion rate as the amount of legal currency spent in the event E by the user A is larger. Thus, on the assumption that a user A whose amount of legal currency spent in the event E is large has a strong tendency to become the well-being state, the monetary value of virtual currency generated by such a user A is adequately raised. In particular, the virtual currency management device 50 is operable to set the virtual currency conversion rate based on the cumulative amount or average amount of respective amounts of legal currency spent in a plurality of events E, so that it becomes possible to set the virtual currency conversion ratio while adequately taking into account an overall tendency for the user A to spend legal currency in the plurality of events E.

In this embodiment, the virtual currency management device 50 is operable to increase the virtual currency conversion rate as the accuracy of estimation of the WA value as the basis for generating the virtual currency is higher. Thus, on the assumption that a user A who willingly takes a behavior for estimating the WB value in an event E (i.e., a user A who is cooperative in estimating the WB value is considered to have a strong tendency to become the well-being state, the monetary value of virtual currency generated by such a user can be adequately raised. In particular, the virtual currency management device 50 is operable to set the virtual currency conversion rate based on the average amount of respective amounts of the WB values estimated in a plurality of events E, so that it becomes possible to set the virtual currency conversion ratio while adequately taking into account an overall tendency of the WB value estimation accuracy in the plurality of events E.

In this embodiment, the virtual currency management device 50 is operable to give a given benefit to a user A or user group A1 who generated virtual currency bought by the third party. This makes it possible to encourage the user A or user group A1 to be motivated to participated in an event E, and encourage a consumption behavior in the event E.

In this embodiment, the virtual currency management device 50 is operable, until a given time period elapses since the third party bought virtual currency, to restrict buying of the virtual currency, so that it becomes possible to suppress selling and buying of virtual currency by the third party on a short-term basis, thereby ensuring a long-term investment (financial support) by the third party.

In the above embodiments, an investor/investors I is/are exemplified as the third party. However, the present invention is not limited thereto. In another embodiment, the third party may be a given administrative agency. In this case, for example, subsidy (administrative subsidy) invests in the financial organization H to buy virtual currency.

LIST OF REFERENCE SIGNS

S: life activation system
1: vehicle
3: communication line
10: measurement device

20: mobile terminal
30: management device
50: virtual currency management device

## Claims

1. A virtual currency management device comprising at least a computer, wherein the virtual currency management device is configured to:

   estimate a well-being value as a mental activity level of a user who participates in a give event;
   store an amount of legal currency spent in the event by the user;
   generate virtual currency based on the estimated well-being value;
   store and manage the generated virtual currency in association with the user;
   perform processing for selling the managed virtual currency to a third party, and buying out the virtual currency of the third party; and
   set a conversion rate between the virtual currency and the legal currency to be applied when the third party buys or sells the virtual currency, such that a monetary value of the virtual currency increases relative to the legal currency as the stored amount of the legal currency is larger.

2. The virtual currency management device according to claim 1, wherein the conversion rate is set based on a cumulative amount or average amount of the legal currency spent in a plurality of events by the user.

3. The virtual currency management device according to claim 1 or 2, which is configured to store an estimation accuracy of the well-being value, and set the conversion rate such that the monetary value of the virtual currency increases relative to the legal currency as the stored estimation accuracy of the well-being value is higher.

4. The virtual currency management device according to claim 3, wherein the conversion rate is set based on an average of the estimation accuracy of the well-being value estimated in a plurality of events.

5. The virtual currency management device according to claim 3 or 4, which is configured to be capable of acquiring a plurality of types of data, for the estimation of the well-being value, and determine that the estimation accuracy of the well-being value is higher as the number of the types of data actually used in the estimation of the well-being value among the plurality of types of data is larger.

6. The virtual currency management device according to claim 5, which is configured to be capable of acquiring, as the plurality of types of data, one or more pieces of biological data indicative of a physiological state of the user during event participation, emotion data based on voice made by the user during event participation, and evaluation data which is a level of a subjective evaluation made by the user for each event.

7. The virtual currency management device according to claim 3 or 4, which is configured to:

   estimate the well-being value, based on at least one selected from the group consisting of one or more pieces of biological data indicative of a physiological state of the user during event participation, emotion data based on voice made by the user during event participation, and evaluation data which is a level of a subjective evaluation made by the user for each event, and
   determine that the estimation accuracy of the well-being value estimated based on the evaluation data is higher than the estimation accuracy of the well-being value estimated based on the emotion data or the biological data.

8. The virtual currency management device according to any one of claims 1 to 7, which is configured to set the conversion rate, with respect to each virtual currency of a single user, or with respect to each virtual currency of a given group including a plurality of users.

9. A virtual currency management method executed by a computer, the method comprising the steps of:

   estimating a well-being value as a mental activity level of a user who participates in a give event;
   storing an amount of legal currency spent in the event by the user;
   generating virtual currency based on the estimated well-being value;

storing and managing the generated virtual currency in association with the user;
performing processing for selling the managed virtual currency to a third party, and buying out the virtual currency of the third party; and
setting a conversion rate between the virtual currency and the legal currency to be applied when the third party buys or sells the virtual currency, such that a monetary value of the virtual currency increases relative to the legal currency as the stored amount of the legal currency is larger.

# FIG.1

FIG.2

# FIG.3A

# FIG.3B

# FIG.4

# FIG.5

```
        ( START )
            │
            ▼
┌─────────────────────────┐
│ Transmit inquiry information │  ～S11
└─────────────────────────┘
            │
            ▼
┌─────────────────────────┐
│ Receive evaluation information │  ～S12
└─────────────────────────┘
            │
            ▼
┌─────────────────────────┐
│ Calculate WB estimate values │  ～S13
└─────────────────────────┘
            │
            ▼
┌─────────────────────────┐
│ Calculate other WB estimate values │  ～S14
└─────────────────────────┘
            │
            ▼
┌─────────────────────────┐
│ Calculate index value │  ～S15
└─────────────────────────┘
            │
            ▼
┌─────────────────────────┐
│ Calculate WB value │  ～S16
└─────────────────────────┘
            │
            ▼
        ( END )
```

# FIG.6

# FIG.7

50

Virtual Currency Management Device

50c
Communication circuit

50d
Input-output device

50e
Display device

Processor —50a

Memory —50b

# FIG.8

START

S21 — Acquire amount of legal currency spent in event

S22 — Acquire WB value estimation value

S23 — Set conversion rate between virtual currency and legal currency

S24 — Execute conversion processing between virtual currency and legal currency, according to conversion rate

RETURN

## INTERNATIONAL SEARCH REPORT

| International application No. |
| --- |
| PCT/JP2019/040233 |

| A. CLASSIFICATION OF SUBJECT MATTER |
| --- |
| Int.Cl. G06Q20/06(2012.01)i, G06Q20/38(2012.01)i |
| |
| According to International Patent Classification (IPC) or to both national classification and IPC |

| B. FIELDS SEARCHED |
| --- |
| Minimum documentation searched (classification system followed by classification symbols) |
| Int.Cl. G06Q20/06, G06Q20/38 |

| Documentation searched other than minimum documentation to the extent that such documents are included in the fields searched | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

| C. DOCUMENTS CONSIDERED TO BE RELEVANT | | |
| --- | --- | --- |
| Category* | Citation of document, with indication, where appropriate, of the relevant passages | Relevant to claim No. |
| A | JP 2018-128740 A (JAPAN POST BANK CO., LTD.) 16 August 2018, entire text (Family: none) | 1–9 |
| A | JP 2018-81426 A (MITSUBISHI RESEARCH INSTITUTE, INC.) 24 May 2018, entire text (Family: none) | 1–9 |
| A | KR 10-2011-0127029 A (UNIV KYUNG HEE UNIV IND COOP GROUP) 24 November 2011, entire text (Family: none) | 1–9 |

| ☐ Further documents are listed in the continuation of Box C. | ☐ See patent family annex. |
| --- | --- |

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 10 December 2019 (10.12.2019) | 17 December 2019 (17.12.2019) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**EP 3 852 039 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6352463 B

- JP 5871347 B